# EUROPEAN PATENT SPECIFICATION

(11) **EP 3 953 705 B1**
(45) Date of publication and mention of the grant of the patent: **24.04.2024**
(21) Application number: 20708639.8
(22) Date of filing: 14.02.2020
(51) Int. Cl.: G01N 33/24, A01B 63/00, A01B 79/00, E01C 19/28, E02D 1/02

(54) **APPARATUS AND METHODS FOR MEASURING SOIL CONDITIONS**
VORRICHTUNG UND VERFAHREN ZUR MESSUNG VON BODENBEDINGUNGEN
APPAREIL ET PROCÉDÉS DE MESURE DE CONDITIONS DE SOL

(30) Priority: 11.04.2019 US 201962832621 P
(43) Date of publication of application: 16.02.2022
(73) Proprietor: Precision Planting LLC, Tremont, IL 61568 (US)
(72) Inventor: STOLLER, Jason, Tremont, Illinois 61568 (US); WILDERMOUTH, Paul, Tremont, Illinois 61568 (US); SWANSON, Todd, Tremont, Illinois 61568 (US)
(74) Representative: AGCO Intellectual Property Department
(86) International application number: PCT/IB2020/051243
(87) International publication number: WO 2020/208431

(56) References cited:
- US-A- 6 041 582
- US-A1- 2005 199 045
- US-B2- 6 834 550
- JUAN AGÜERA ET AL: "Design of a Soil Cutting Resistance Sensor for Application in Site-Specific Tillage", SENSORS, vol. 13, no. 5, 10 May 2013 (2013-05-10), pages 5945-5957, XP055626009, DOI: 10.3390/s130505945
- Fatemeh Rahimi Ajdadi ET AL: "A REVIEW ON THE SOIL COMPACTION MEASUREMENT SYSTEMS", 1st International and 5th National Conference on Organic vs. Conventional Agriculture, 16 August 2017 (2017-08-16), pages 1-5, XP055687349, Retrieved from the Internet: URL:https://www.researchgate.net/publicati on/323218205_A_REVIEW_ON_THE_SOIL_COMPACTI ON_MEASUREMENT_SYSTEMS [retrieved on 2020-04-20]
- LUI W ET AL: "DEVELOPMENT OF A TEXTURE/SOIL COMPACTION SENSOR", PROCEEDINGS INTERNATIONAL CONFERENCE ON PRECISION AGRICULTURE, XX, XX, 23 June 1996 (1996-06-23), pages 617-630, XP001133771,

## Description

### FIELD

Embodiments of the present disclosure relate to measurement of soil conditions. More particularly, embodiments of the present invention relate to apparatus and methods for measuring soil compaction in conjunction with planting.

### BACKGROUND

Crop yields are affected by a variety of factors, such as seed placement, soil quality, weather, irrigation, and nutrient applications. Soil compaction affects how seeds are placed, as well as how water and fertilizer permeates the soil. Thus, tests have been developed to measure soil compaction in agricultural fields. As used herein, the term "soil compaction" is a measure of the volume of solid material within a given volume of soil as compared to the volume of liquid or gases *(e.g.,* in pores between particles of solid material). Soil compaction is proportional to soil density of dry soil. Information about soil compaction is valuable because it assists farmers with determining how deep to plant seeds, how much to water and fertilizer to apply, *etc.* Furthermore, soil compaction is related to the force required to break through soil so that seeds can be planted below the surface. Crop yield can also be affected by soil compaction. Significant changes in soil compaction or soil density in the soil profile of the root zone of a plant can adversely affect crop yield. For example, a large change in soil compaction may cause roots to change direction when they reach the soil with high compaction. Soil compaction typically varies throughout a field and with depth beneath the surface. A no-till field could have a higher soil density or soil compaction compared to tilled field, all other variables being equal, but the density and compaction of the no-till field could still be within acceptable ranges. Therefore, the no-till field may still produce similar or better crop yield than the tilled field if rapid and significant soil density changes are minimized. Information about the density and compaction can help farmers make decisions about whether tilling is required or if tillage depth should increase or decrease.

Methods of measuring soil compaction are described in U.S. Patent 6,834,550, "Soil Profile Force Measurement Using an Instrumented Tine," issued December 28, 2004.

J. Agiiera et al, in "Design of a Soil Cutting Resistance Sensor for Application in Site-Specific Tillage", Sensors 2013, 13, 5945-5957, discloses a soil strength sensor for measuring soil cutting resistance at various depths while traversing a field.

### BRIEF SUMMARY

In accordance with a first aspect of the invention, an apparatus for measuring a soil condition includes a plurality of elongate beams mounted on opposing sides of a shank and arranged at different heights along the shank, and a plurality of load cells. Each load cell of the plurality is coupled to the shank and to a beam of the plurality such that a horizontal force on the beam induces the load cell to generate a signal corresponding to a force on the beam.

In accordance with a second aspect of the invention, a method of measuring a property of soil includes dragging at least a portion of a shank through soil. The shank carries a plurality of elongate beams mounted on opposing sides of the shank and arranged at different heights. The method also includes inducing a force on each of a plurality of load cells related to horizontal forces on the plurality of beams, wherein each load cell is coupled to the shank and to a beam of the plurality. The method includes generating signals with the load cells. The signals are related to the forces on the load cells. The method may be used to measure soil compaction and/or identify a compaction layer.

### BRIEF DESCRIPTION OF THE DRAWINGS

While the specification concludes with claims particularly pointing out and distinctly claiming what are regarded as embodiments of the present disclosure, various features and advantages of embodiments of the disclosure may be more readily ascertained from the following description of example embodiments when read in conjunction with the accompanying drawings, in which:
FIG. 1 is a simplified side view of an apparatus for measuring a soil condition;
FIG. 2 is a simplified side view of the apparatus of FIG. 1 in a configuration for transport;
FIG. 3 is a simplified front view of another apparatus for measuring a soil condition;
FIG. 4 is a simplified front view of an embodiment of an apparatus for measuring a soil condition;
FIG. 5 is a simplified side view of a portion of the apparatus of FIG. 4;
FIG. 6 is a simplified top view of a portion of the apparatus of FIG. 4;
FIG. 7 is a simplified flow chart illustrating an example method of operating the apparatuses shown in FIGS. 1 through 6; and
FIG. 8 is a simplified graph of soil compaction, depicted as the force required to move an implement through soil as a function of depth.

### DETAILED DESCRIPTION

The illustrations presented herein are not actual views of any measuring tool or portion thereof, but are merely idealized representations that are employed to describe example embodiments of the present disclosure. Additionally, elements common between figures may retain the same numerical designation.

The following description provides specific details of embodiments of the present disclosure in order to provide a thorough description thereof. However, a person of ordinary skill in the art will understand that the embodiments of the disclosure may be practiced without employing many such specific details. Indeed, the embodiments of the disclosure may be practiced in conjunction with conventional techniques employed in the industry. In addition, the description provided below does not include all elements to form a complete structure or assembly. Only those process acts and structures necessary to understand the embodiments of the disclosure are described in detail below. Additional conventional acts and structures may be used. Also note, the drawings accompanying the application are for illustrative purposes only, and are thus not drawn to scale.

As used herein, the terms "comprising," "including," "containing," "characterized by," and grammatical equivalents thereof are inclusive or open-ended terms that do not exclude additional, unrecited elements or method steps, but also include the more restrictive terms "consisting of" and "consisting essentially of" and grammatical equivalents thereof.

As used herein, the term "may" with respect to a material, structure, feature, or method act indicates that such is contemplated for use in implementation of an embodiment of the disclosure, and such term is used in preference to the more restrictive term "is" so as to avoid any implication that other, compatible materials, structures, features, and methods usable in combination therewith should or must be excluded.

As used herein, the term "configured" refers to a size, shape, material composition, and arrangement of one or more of at least one structure and at least one apparatus facilitating operation of one or more of the structure and the apparatus in a predetermined way.

As used herein, the singular forms following "a," "an," and "the" are intended to include the plural forms as well, unless the context clearly indicates otherwise.

As used herein, the term "and/or" includes any and all combinations of one or more of the associated listed items.

As used herein, spatially relative terms, such as "beneath," "below," "lower," "bottom," "above," "upper," "top," "front," "rear," "left," "right," and the like, may be used for ease of description to describe one element's or feature's relationship to another element(s) or feature(s) as illustrated in the figures. Unless otherwise specified, the spatially relative terms are intended to encompass different orientations of the materials in addition to the orientation depicted in the figures.

As used herein, the term "substantially" in reference to a given parameter, property, or condition means and includes to a degree that one of ordinary skill in the art would understand that the given parameter, property, or condition is met with a degree of variance, such as within acceptable manufacturing tolerances. By way of example, depending on the particular parameter, property, or condition that is substantially met, the parameter, property, or condition may be at least 90.0% met, at least 95.0% met, at least 99.0% met, or even at least 99.9% met.

As used herein, the term "about" used in reference to a given parameter is inclusive of the stated value and has the meaning dictated by the context *(e.g.,* it includes the degree of error associated with measurement of the given parameter).

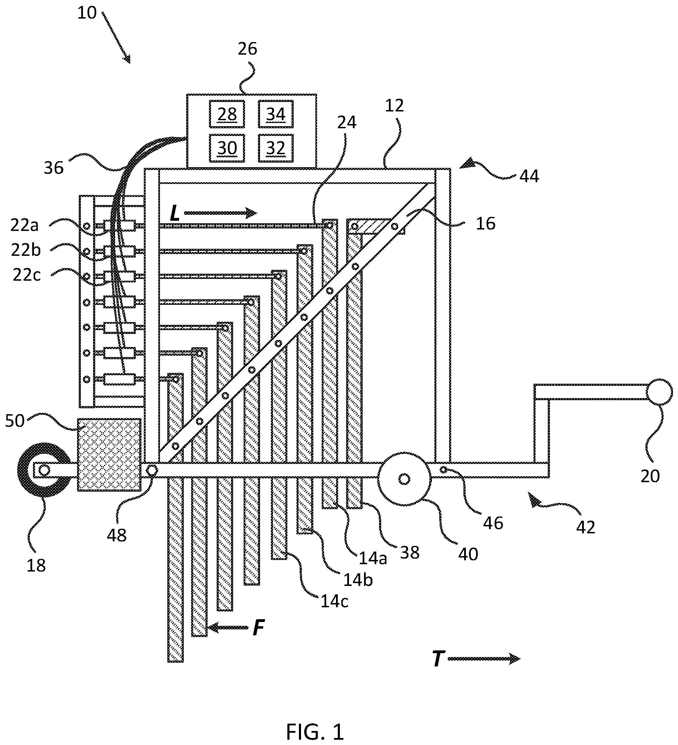

FIG. 1 is a simplified side view of an apparatus 10 for measuring a soil condition. In particular, the apparatus 10 may be used to measure a force required to break through soil as a function of depth, which may be used to calculate soil compaction, or which may be used as a proxy for soil compaction.

The apparatus 10 includes a frame 12 and a plurality of beams 14 (indicated 14a, 14b, and 14c, with others unlabeled for simplicity) carried by the frame 12. The beams 14 may be elongate, having a length much greater than a width or thickness. The beams 14 are arranged such that a lower extent of each beam 14 differs from other beams 14. The beams 14 may be pivotally coupled to the frame 12, such as by a diagonal member 16 of the frame 12. The apparatus 10 may also include one or more wheels 18 (*e.g.,* a single wheel, two or more wheels attached to a common axle, wheels attached to different axles, *etc*.) and a tow hitch 20 to support the frame 12 and facilitate travel of the apparatus 10 over an agricultural field.

The apparatus 10 may also include a plurality of load cells 22 (indicated 22a, 22b, and 22c, with others unlabeled for simplicity), each coupled to frame 12 and to an upper portion of one of the beams 14. For example, the load cell 22a is pivotally coupled to the beam 14a by a connector 24, such as a rod, beam, *etc.* Thus, when a horizontal force (*i.e.,* left or right, in the orientation shown in FIG. 1) acts on the lower extent of the beams 14, the beams 14 may exert an opposite force on the load cells 22 (though the magnitude of the force may not be equal to the force on the lower extent of the beams 14, as dictated by the length of the beams 14 above and below the connection to the diagonal member 16 of the frame 12). The load cells 22 may be strain gauges, load pins, platform cells, bending beams, or any other type of load cell. In general, the apparatus 10 moves forward in the direction of travel ***T*** shown in FIG. 1. If the lower extent of a beam 14 is below ground, the ground exerts a force ***F*** on the beam 14 in the direction opposite the direction of travel ***T**.* The beam 14 exerts a load ***L*** on the corresponding load cell 22 (depicted for clarity adjacent the connector 24 connecting the load cell 22a to the beam 14a). In certain embodiments, other devices may be used to measure the load ***L*** on the beam 14. For example, a hall-effect sensor may be configured to measure deflection of the beam 14, which deflection may be used to calculate the load ***L*** (or may be used to calculate the soil conditions directly without calculating the load ***L***).

Though in the configuration shown in FIG. 1, the loads ***L*** on the load cells 22 are in tension (*i.e.,* pulling), the load cells 22 may be configured such that the loads ***L*** on the load cells 22 are in compression, such as by positioning the load cells 22 below the pivot point of the beams 14 or in front of the beams 14.

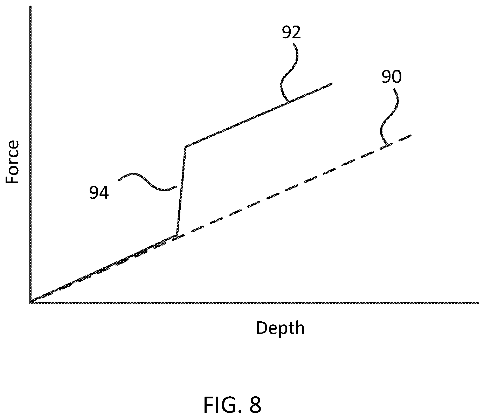

The apparatus 10 may also include a computer 26 that has a receiver 28, a processor 30, a computer-readable medium 32 *(e.g.,* a flash drive, CD-R, DVD-R, applicationspecific integrated circuit (ASIC), field-programmable gate array (FPGA), a platter of a hard disk drive, *etc*.), and/or a transmitter 34. The receiver 28 is configured to receive electrical signals from the load cells 22 via, for example, wires 36. In some embodiments, the load cells 22 may transmit electromagnetic signals wirelessly to the receiver 28, and thus, the wires 36 may be omitted. The processor 30 may calculate a property of the soil using the signals from the receiver 28. In particular, the processor 30 may calculate a load profile as a function of depth. For example, as shown in FIG. 8, in an ideal soil load profile 90 of uncompacted soil, the cumulative force on an implement passing through the soil increases uniformly with depth. In a real soil load profile 92, the cumulative force on an implement may increase relatively uniformly for a certain depth, after which the force increases drastically at the beginning of a compaction layer, indicated by the rise 94 shown in FIG. 8. After breaking through the compaction layer, the force may rise at a rate similar to the rate above the compaction layer.

The processor 30 may store data on the computer-readable medium 32 and/or retrieve instructions encoded on the computer-readable medium 32. The transmitter 34 may transmit the load profile, the data from the load cells 22, or any other information to a remote location, such as a cab of a vehicle towing the apparatus 10, another vehicle, a remote operator, *etc.* The transmitter 34 may transmit information via wired or wireless communication.

The apparatus 10 may also include another beam 38 that is structured and positioned to lead at least one of the beams 14 when the apparatus 10 moves in the direction of travel ***T**.* The beam 38 may have approximately the same dimensions as the beam 14 it leads (*i.e.,* beam 14a in FIG. 1). However, the beam 38 may be connected to the frame 12 in such a way that does not exert a force on a load cell. For example, and as shown in FIG. 1, the beam 38 may be rigidly attached to the diagonal member 16 of the frame 12.

The apparatus 10 may also include a coulter 40 secured to the frame 12 leading the beams 14. The coulter 40 may be set at a depth such that it pushes aside loose soil atop a compacted layer. In some embodiments, the coulter 40 may be dynamically adjusted during use, such as using springs or other biasing elements. The coulter 40 may cut residue or shift the residue outward to prevent dragging residue with soil (which dragging may negatively affect the consistency of soil measurement). In other embodiments, a floating or fixed row cleaner may be used in place of the pictured coulter 40.

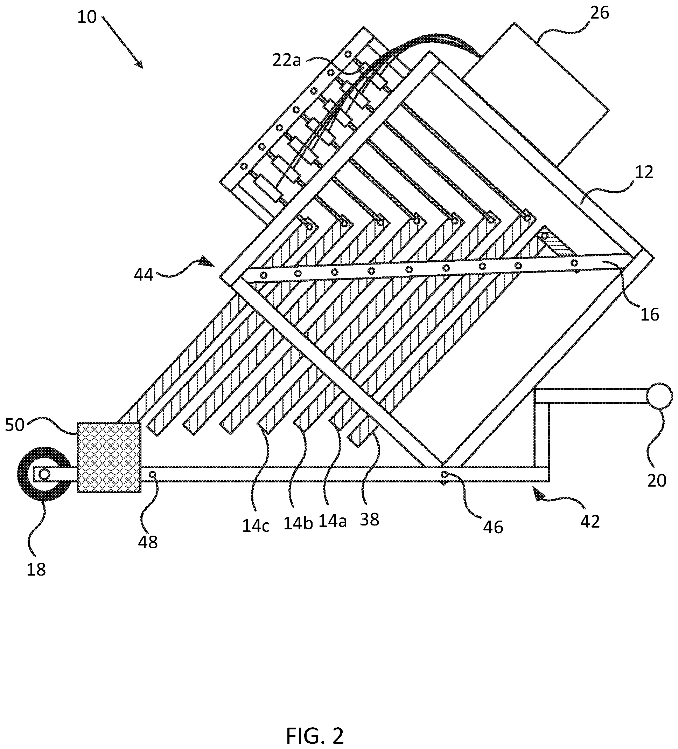

The frame 12 may include a base 42 and a superstructure 44 coupled together. The base 42 may include or carry the wheels 18 and tow hitch 20, if present. The superstructure 44 may include the diagonal member 16 and supports for the load cells 22. The computer 26 is depicted on the superstructure 44, but may alternatively be carried by the base 42 by rerouting the wires 36 (if present). The superstructure 44 may be coupled to the base 42 by a pivot point 46 and a detachable connector 48. The pivot point 46 may include, for example, a pin or similar mechanism. The detachable connector 48 may include one or more bolts. After the detachable connector 48 is disconnected, and as shown in FIG. 2, the superstructure 44 may be pivoted on the pivot point 46 such that the beams 14 swing upward, away from the ground. FIG. 2 also shows that the coulter 40 has been removed, but it may also be pivoted or adjusted upward to prevent it from contacting the ground. The apparatus 10 may then travel along the ground without disturbing the ground with the beams 14. In particular, this configuration is useful for transport of the apparatus 10 to the field where it will be used, such as along a public roadway or from a storage location to a field.

The apparatus 10 may include one or more weights 50 to help keep the apparatus 10 and the beams 14 at a constant position with respect to the surface of the ground. The weights 50 may also be used to adjust the center of gravity of the apparatus 10.

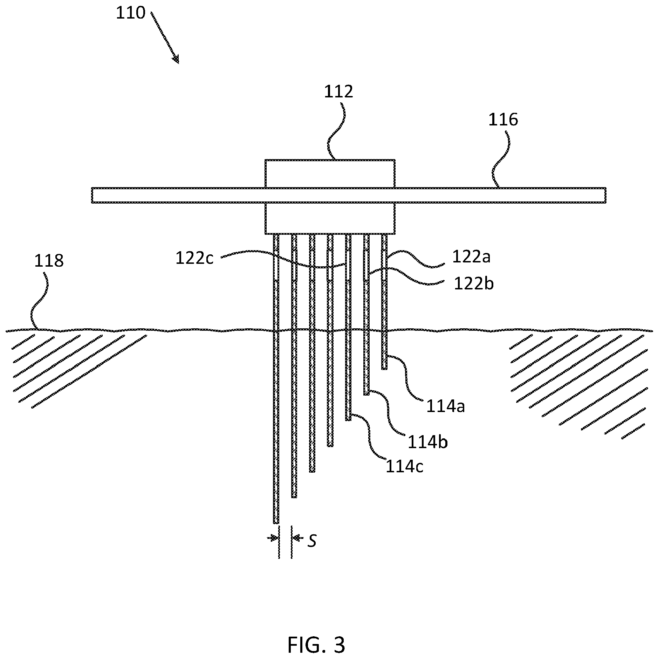

FIG. 3 illustrates an apparatus 110 for measuring a soil condition. The apparatus 110 is shown in front view, and includes a frame 112 carried by a toolbar 116 of a vehicle (not pictured). Beams 114 (indicated 114a, 114b, and 114c, with others unlabeled for simplicity) are attached to the frame 112. Load cells 122 (indicated 122a, 122b, and 122c, with others unlabeled for simplicity) are configured to detect forces applied to the beams 114. The beams 114 are shown extending below the surface of the ground 118, such that when the apparatus 110 moves along the surface of the ground 118, the beams 114 are deflected based on the resistance of the ground 118. The beams 114 that extend deeper into the ground (*e.g.,* 114c) typically experience greater deflection than beams 114 that do not extend as deep (*e.g.,* 114a, 114b). Spacing ***S*** between the beams 114 may be selected such that each beam 114 does not disturb the neighboring beam(s) 114 when the beams 114 are dragged through the ground 118. That is, the ground 118 disturbed by one beam 114 may not significantly affect the ground 118 adjacent beams 114. For example, the spacing ***S*** may be at least about 1 cm, at least about 2 cm, at least about 5 cm, or even at least about 10 cm.

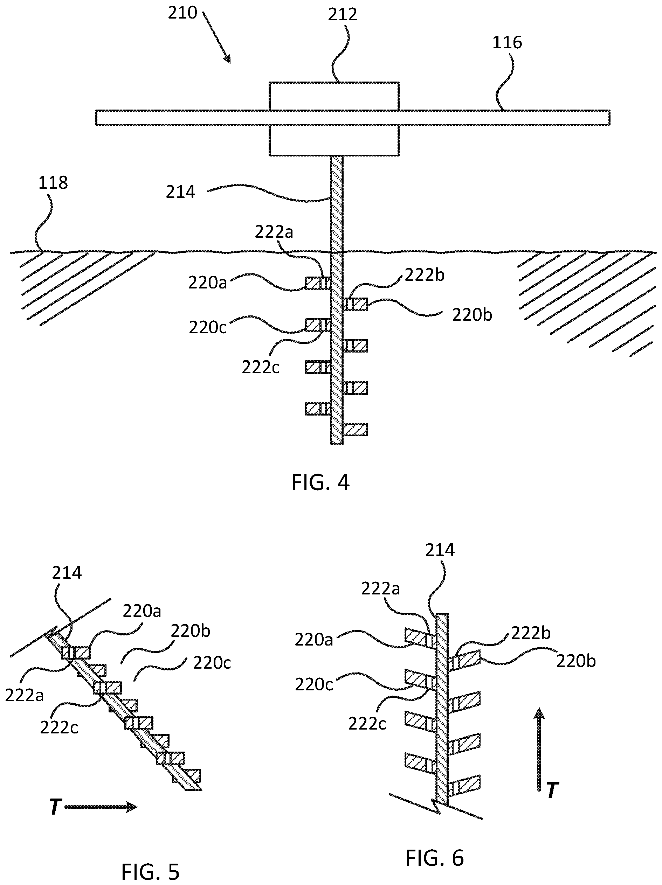

FIG. 4 illustrates another embodiment of an apparatus 210 for measuring a soil condition. The apparatus 210 is shown in front view, and includes a frame 212 carried by a toolbar 116 of a vehicle (not pictured). The frame 212 includes a shank 214, which carries beams 220 (indicated 220a, 220b, and 220c, with others unlabeled for simplicity). The beams 220 are mounted on opposing sides of the shank 214. Load cells 222 (indicated 222a, 222b, and 222c, with others unlabeled for simplicity) are configured to detect forces applied to the beams 220. The beams 220 are shown below the surface of the ground 118, such that when the apparatus 210 moves along the surface of the ground 118, the beams 220 are deflected based on the resistance of the ground 118. The beams 220 that are located deeper into the ground (*e.g.,* 220c) typically experience greater deflection than beams 220 that are not as deep (*e.g.,* 220a, 220b).

FIG. 5 is a side view of the portion of the apparatus 210 of FIG. 4 that is below ground. As shown, the shank 214 may be a diagonal member, angled forward such that the lowest beams 220 lead the higher beams 220 as the apparatus 210 moves in the direction of travel ***T.***

FIG. 6 is a top view of the portion of the apparatus 210 of FIG. 4 that is below ground. As shown, the beams 220 may each be angled forward such that the portions of the beams 220 farthest from shank 214 lead portions of the beams 220 closest to the shank 214 as the apparatus 210 moves in the direction of travel ***T**.*

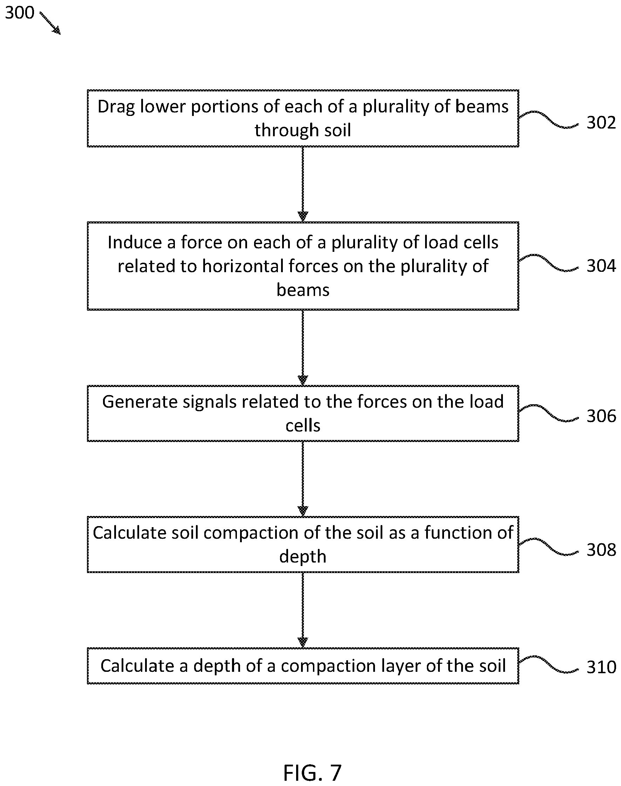

FIG. 7 is a simplified flow chart illustrating an example method 300 of measuring a property of soil using the apparatus 10, 110, or 210 shown in FIGS. 1-6 and described above. Some operations shown in FIG. 7 are optional, and a person having ordinary skill in the art could select the order of operations to fit operational needs. The operations shown in FIG. 7 may be performed at substantially the same time, and may be performed continuously while operating the apparatus 10, 110, or 210. The flow chart in FIG. 7 is not intended to be limiting.

The method 300 depicted includes, as shown in element 302, dragging lower portions of each of a plurality of beams through soil. The beams are carried by a frame and arranged such that a lower extent of the beams differs from one another. The dragging may be performed by towing the frame over the soil with a vehicle. In some embodiments, the dragging may include dragging the beams in a common plane parallel to a direction of travel of the beams.

The method 300 also includes, as shown in element 304, inducing a force on each of a plurality of load cells related to a horizontal force on the lower portions of the plurality of beams. Each load cell is coupled to the frame and a portion of a beam of the plurality.

As shown in element 306, the method may include generating signals with the load cells. The signals are related to the forces on the load cells.

As shown in element 308, the method may include calculating soil compaction of the soil as a function of depth. For example, the method may include calculating a force required to move an implement through soil of a certain depth, and the data may be a part of the load profile 92 shown in FIG. 8.

Furthermore, in some embodiments and as shown in element 310, the method may include calculating a depth of a compaction layer of the soil. For example, the rise 94 in the load profile 92 shown in FIG. 8 may indicate the depth of the compaction layer.

The apparatuses 10, 110, 210 disclosed herein may be used in conjunction with planting a field. For example, data may be collected to map the soil conditions of a field before or during a planting operation. In some embodiments, the soil conditions may be measured in real time by an apparatus 10, 110, 210 carried by a vehicle that also carries a planting apparatus. The planting apparatus (*e.g.,* planting depth, downforce, seed population, *etc.*) may be adjusted based on information from the apparatus 10, 110, 210. By measuring the soil compaction and adjusting planting accordingly, the overall yield of the field may be increased because the planting parameters of each portion of the field may be tailored to the soil conditions at that location.

While the present disclosure has been described herein with respect to certain illustrated embodiments, those of ordinary skill in the art will recognize and appreciate that it is not so limited. Rather, many additions, deletions, and modifications to the illustrated embodiments may be made without departing from the scope of the disclosure as hereinafter claimed, including legal equivalents thereof. In addition, features from one embodiment may be combined with features of another embodiment while still being encompassed within the scope as contemplated by the inventors. Further, embodiments of the disclosure have utility with different and various machine types and configurations.

## Claims

1. An apparatus (210) for measuring a soil condition, the apparatus (210) comprising:
a plurality of elongate beams (220a-c) mounted on opposing sides of a shank (214) and arranged at different heights along the shank (214); and
a plurality of load cells (222a-c), each load cell (222a-c) of the plurality coupled to the shank (214) and to a beam (220a-c) of the plurality such that a horizontal force on the beam (220a-c) induces the load cell (222a-c) to generate a signal corresponding to a force on the beam (220a-c).

2. The apparatus (210) of claim 1, further comprising a receiver (28) in communication with the load cells (222a-c), the receiver (28) configured to receive the signals from the load cells (222a-c).

3. The apparatus (210) of claim 2, further comprising a processor (30) configured to calculate a property of soil through which at least portions of the beams (220a-c) pass.

4. The apparatus (210) of claim 3, further comprising a transmitter (34) configured to transmit the property of the soil.

5. The apparatus (210) of any one of claims 1 through 4, wherein the shank (214) is carried by a frame (212), and further comprising a tow hitch coupled to the frame (212).

6. The apparatus (210) of any one of claims 1 through 4, wherein the plurality of beams (220a-c) are configured to be disposed entirely below ground (118) when the apparatus (210) is moved along the ground (118).

7. A method (300) of measuring a property of soil, the method comprising:
dragging (302) at least a portion of a shank (214) through soil, the shank (214) carrying a plurality of elongate beams (220a-c) mounted on opposing sides of the shank (214) and arranged at different heights;
inducing (304) a force on each of a plurality of load cells (222a-c) related to horizontal forces on the plurality of beams (220a-c), wherein each load cell (222a-c) is coupled to the shank (214) and to a beam (220a-c) of the plurality; and
generating (306) signals with the load cells (222a-c), the signals related to the forces on the load cells (220a-c).

8. The method (300) of claim 7, further comprising calculating (308) soil compaction of the soil as a function of depth based at least in part on the signals.

9. The method (300) of claim 8, further comprising calculating (310) a depth of a compaction layer of the soil based at least in part on the signals.

10. The method (300) of any one of claims 7 through 9, wherein dragging at least a portion of a shank (214) through soil comprises towing the shank (214) through the soil with a vehicle.

11. The method (300) of any one of claims 7 through 9, wherein dragging at least a portion of a shank (214) through soil comprises orienting the shank (214) such that a first beam (220a) of the plurality leads a second beam (220b) of the plurality in a direction of travel (T) of the shank (214), the first beam (220a) deeper in the soil than the second beam (220b).

12. The method (300) of any one of claims 7 through 9, wherein dragging at least a portion of a shank (214) through soil comprises orienting the shank (214) such that a portion of a beam (220a-c) of the plurality distal from the shank (214) leads another portion of the beam (220a-c) connected to the shank (214) in a direction of travel (T) of the shank (214).

## Patentansprüche

1. Vorrichtung (210) zum Messen eines Bodenzustands, wobei die Vorrichtung (210) aufweist:
eine Mehrzahl von länglichen Stäben (220a-c), welche auf gegenüberliegenden Seiten eines Schafts (214) befestigt und auf unterschiedlichen Höhen entlang des Schafts (214) angeordnet sind; und
eine Mehrzahl von Kraftaufnehmern (222a-c), wobei jeder Kraftaufnehmer (222a-c) der Mehrzahl von Kraftaufnehmern (222a-c) so mit dem Schaft (214) und mit einem Stab (220a-c) der Mehrzahl von Stäben (220a-c) gekoppelt ist, dass eine horizontale Kraft auf den Stab (220a-c) den Kraftaufnehmer (222a-c) veranlasst, ein zu einer Kraft auf den Stab (220a-c) korrespondierendes Signal zu generieren.

2. Vorrichtung (210) nach Anspruch 1, weiterhin mit einem in Kommunikation mit den Kraftaufnehmern (222a-c) stehenden Empfänger (28), wobei der Empfänger (28) eingerichtet ist, die Signale von den Kraftaufnehmern (222a-c) zu empfangen.

3. Vorrichtung (210) nach Anspruch 2, weiterhin mit einem Prozessor (30), welcher eingerichtet ist, eine Eigenschaft eines Bodens, durch welchen zumindest Teile der Stäbe (220a-c) hindurchtreten, zu berechnen.

4. Vorrichtung (210) nach Anspruch 3, weiterhin mit einem Sender (34), welcher eingerichtet ist, die Eigenschaft des Bodens zu übermitteln.

5. Vorrichtung (210) nach einem der Ansprüche 1 bis 4, wobei der Schaft (214) von einem Rahmen (212) getragen wird, und die Vorrichtung weiterhin eine an den Rahmen (212) gekoppelte Anhängerkupplung aufweist.

6. Vorrichtung (210) nach einem der Ansprüche 1 bis 4, wobei die Mehrzahl von Stäben (220a-c) eingerichtet ist, vollständig unter einer Bodenoberfläche (118) angeordnet zu werden, wenn die Vorrichtung (210) entlang der Bodenoberfläche (118) bewegt wird.

7. Verfahren (300) zum Messen einer Eigenschaft eines Bodens, wobei das Verfahren aufweist:
Ziehen (302) zumindest eines Teils eines Schafts (214) durch einen Boden, wobei der Schaft (214) eine Mehrzahl von länglichen Stäben (220a-c) trägt, welche auf gegenüberliegenden Seiten des Schafts (214) montiert und auf unterschiedlichen Höhen angeordnet sind;
Einleiten (304) einer Kraft auf jeden einer Mehrzahl von Kraftaufnehmern (222a-c) bezogen auf horizontale Kräfte auf die Mehrzahl von Stäben (220a-c) wobei jeder Kraftaufnehmer (222a-c) mit dem Schaft (214) und einem Stab (220a-c) der Mehrzahl gekoppelt ist; und
Generieren (306) von Signalen mit den Kraftaufnehmern (222a-c), wobei sich die Signale auf die Kräfte auf die Kraftaufnehmer (220a-c) beziehen.

8. Verfahren (300) nach Anspruch 7, weiterhin mit Ermitteln (308) einer Bodenverdichtung des Bodens als Funktion der Tiefe basierend zumindest teilweise auf den Signalen.

9. Verfahren (300) nach Anspruch 8, weiterhin mit Ermitteln (310) einer Tiefe einer Verdichtungsschicht des Bodens basierend zumindest teilweise auf den Signalen.

10. Verfahren (300) nach einem der Ansprüche 7 bis 9, wobei das Ziehen zumindest eines Teils eines Schafts (214) durch einen Boden ein Schleppen des Schafts (214) durch den Boden mit einem Fahrzeug aufweist.

11. Verfahren (300) nach einem der Ansprüche 7 bis 9, wobei das Ziehen zumindest eines Teils eines Schafts (214) durch einen Boden ein Orientieren des Schafts (214) aufweist, derart dass ein erster Stab (220a) der Mehrzahl einem zweiten Stab (220b) der Mehrzahl in einer Fortbewegungsrichtung (T) des Schafts (214) vorläuft, wobei der erste Stab (220a) tiefer in dem Boden ist als der zweite Stab (220b).

12. Verfahren (300) nach einem der Ansprüche 7 bis 9, wobei das Ziehen zumindest eines Teils eines Schafts (214) durch einen Boden ein Orientieren des Schafts (214) aufweist, derart dass ein dem Schaft (214) ferner Teil eines Stabs (220a-c) der Mehrzahl einem anderen mit dem Schaft (214) verbundenen Teil des Stabs (220a-c) in einer Fortbewegungsrichtung (T) des Schafts (214) vorläuft.

## Revendications

1. Appareil (210) de mesure d'une condition de sol, l'appareil (210) comprenant :
une pluralité de poutres allongées (220a-c) montées sur des côtés opposés d'une tige (214) et agencées à des hauteurs différentes le long de la tige (214) ; et
une pluralité de cellules de charge (222a-c), chaque cellule de charge (222a-c) de la pluralité étant couplée à la tige (214) et à une poutre (220a-c) de la pluralité de sorte qu'une force horizontale sur la poutre (220a-c) induise la cellule de charge (222a-c) à générer un signal correspondant à une force sur la poutre (220a-c) .

2. Appareil (210) selon la revendication 1, comprenant en outre un récepteur (28) en communication avec les cellules de charge (222a-c), le récepteur (28) étant configuré pour recevoir les signaux depuis les cellules de charge (222a-c).

3. Appareil (210) selon la revendication 2, comprenant en outre un processeur (30) configuré pour calculer une propriété d'un sol à travers lequel passent au moins des parties des poutres (220a-c).

4. Appareil (210) selon la revendication 3, comprenant en outre un émetteur (34) configuré pour transmettre la propriété du sol.

5. Appareil (210) selon l'une quelconque des revendications 1 à 4, dans lequel la tige (214) est portée par un cadre (212), et comprenant en outre une barre d'attelage couplée au cadre (212).

6. Appareil (210) selon l'une quelconque des revendications 1 à 4, dans lequel la pluralité de poutres (220a-c) sont configurées pour être disposées entièrement sous une terre (118) lorsque l'appareil (210) est déplacé le long de la terre (118).

7. Procédé (300) de mesure d'une propriété d'un sol, le procédé comprenant :
le traînage (302) d'au moins une partie d'une tige (214) à travers un sol, la tige (214) portant une pluralité de poutres allongées (220a-c) montées sur des côtés opposés de la tige (214) et agencées à des hauteurs différentes ;
l'induction (304) d'une force sur chacune d'une pluralité de cellules de charge (222a-c) liée à des forces horizontales sur la pluralité de poutres (220a-c), dans lequel chaque cellule de charge (222a-c) est couplée à la tige (214) et à une poutre (220a-c) de la pluralité ; et
la génération (306) de signaux avec les cellules de charge (222a-c), les signaux étant liés aux forces sur les cellules de charge (220a-c).

8. Procédé (300) selon la revendication 7, comprenant en outre le calcul (308) d'un compactage de sol du sol en fonction d'une profondeur sur la base au moins en partie des signaux.

9. Procédé (300) selon la revendication 8, comprenant en outre le calcul (310) d'une profondeur d'une couche de compactage du sol sur la base au moins en partie des signaux.

10. Procédé (300) selon l'une quelconque des revendications 7 à 9, dans lequel le traînage d'au moins une partie d'une tige (214) à travers un sol comprend le remorquage de la tige (214) à travers le sol avec un véhicule.

11. Procédé (300) selon l'une quelconque des revendications 7 à 9, dans lequel le traînage d'au moins une partie d'une tige (214) à travers un sol comprend l'orientation de la tige (214) de sorte qu'une première poutre (220a) de la pluralité dirige une deuxième poutre (220b) de la pluralité dans une direction de déplacement (T) de la tige (214), la première poutre (220a) étant plus profonde dans le sol que la deuxième poutre (220b).

12. Procédé (300) selon l'une quelconque des revendications 7 à 9, dans lequel le traînage d'au moins une partie d'une tige (214) à travers un sol comprend l'orientation de la tige (214) de sorte qu'une partie d'une poutre (220a-c) de la pluralité distale de la tige (214) dirige une autre partie de la poutre (220a-c) reliée à la tige (214) dans une direction de déplacement (T) de la tige (214).
